Europäisches Patentamt

European Patent Office

Office européen des brevets

(19)

(11) Publication number: **0 003 624**
**B1**

(12) **EUROPEAN PATENT SPECIFICATION**

(45) Date of publication of patent specification: **28.07.82**

(21) Application number: **79200049.9**

(22) Date of filing: **25.01.79**

(51) Int. Cl.³: **C 07 C 69/67,**
**C 07 C 67/347**

(54) Process for the preparation of the methyl ester of 4-oxopentane-1-carboxylic acid.

(30) Priority: **27.01.78 NL 7800982**

(43) Date of publication of application:
**22.08.79 Bulletin 79/17**

(45) Publication of the grant of the patent:
**28.07.82 Bulletin 82/30**

(84) Designated Contracting States:
**BE CH DE FR GB IT NL**

(56) References cited:
**FR - A - 2 229 679**
**FR - A - 2 323 673**

(73) Proprietor: **STAMICARBON B.V.**
**Postbus 10**
**NL-6160 MC Geleen (NL)**

(72) Inventor: **Balg, Theodorus**
**Beethovenstraat 12**
**NL-6438 KJ Oirsbeek (NL)**
Inventor: **Cramers, Constant Maria Alphons**
**Burg. Houbenstraat 23**
**NL-6124 BE Obbicht (NL)**

(74) Representative: **Pinckaers, August René et al,**
**OCTROOIBUREAU DSM Postbus 9**
**NL-6160 MA Geleen (NL)**

Courier Press, Leamington Spa, England.

Process for the preparation of the methyl ester of 4-oxopentane-1-carboxylic acid.

The invention relates to a process of preparing the methyl ester of 4-oxopentane-1-carboxylic acid by the liquid phase reaction of acetone with methyl acrylate by using a primary amine and/or Schiff base as a catalyst and in the presence of an acidic compound.

According to this process, which is known from British Patent Specification 1,389,510, the reaction mixture obtained upon completion of said reaction is subjected to a first distillation at atmospheric pressure, to remove overhead unconsumed reactants and catalyst. The distillate containing the catalyst and the unconsumed reactants can be re-used in the preparation of further methyl ester, while the residue remaining from the said distillation and containing the methyl ester is subjected to a further distillation at reduced pressure whereby the methyl ester product is recovered in a substantially pure state.

In the said known process it was found that the yield of methyl ester could be adversely affected if the catalyst and the unconsumed reactants are distilled off at a temperature higher than 195 °C, which occurs when atmospheric pressure is used. However the use of reduced pressure to keep the temperature of the reaction mixture below 195 °C results in a polymeric substance depositing onto the wall of the reactor and stirrer used when the resulting distillate is recycled to the reaction mixture, which substance impeded continuous operation of the process.

By the practice of the present invention both the adverse effect on the yield and the above-mentioned deposit of a polymeric substance is inhibited.

The process according to the invention for preparing the methyl ester of 4-oxopentane-1-carboxylic acid by the liquid phase reaction of acetone with methyl acrylate by using a primary amine and/or Schiff base as a catalyst and in the presence of an acid compound, in which the unconsumed reactants and the catalyst are first distilled from the resulting reaction mixture and said methyl ester is recovered from the remaining residue by a further distillation, is characterized in that the amount of oxygen dissolved in the reaction mixture is maintained at a level below 20 parts by weight per million and the unconsumed reactants and the catalyst are distilled off at a temperature below 195 °C at a pressure not lower than atmospheric in the presence of an inert solvent with a boiling point of between 80 and 195 °C.

Examples of suitable inert solvents are mesityl oxide, mesitylene, toluene, xylenes, decalin, dioxane and cumene. The temperature of the first distillation may be controlled by the amount of inert solvent used. Thus the minimum amount of inert solvent to be added to the reaction mixture when the unconsumed reactants and the catalyst are distilled off is that amount at which the temperature of 195 °C in distillation at atmospheric pressure is just not exceeded. If more than said minimum amount of inert solvent is used, this may lower the maximum temperature during distillation to for example 180 °C. The addition of such an amount that said maximum temperature is reduced further than 160 °C offers no practical advantage. After the unconsumed reactants and the catalyst have been distilled off, the inert solvent can be recovered from the remaining residue by a distillation.

The inert solvent used is preferably mesityl oxide, since it is formed as a by-product in the process according to the invention by condensation of acetone to diacetone alcohol and subsequent elimination of water, and thus has to be removed in the distillation of the residue that remains upon removal of the unconsumed reactants and the catalyst by distillation and from which the methyl ester is recovered. The mesityl oxide obtained in said further distillation of the residue is substantially pure and is suitable as such for use as the inert solvent. The distillation of the residue to recover the inert solvent and the methyl ester can be effected at reduced pressure without difficulty.

The required low oxygen content of the reaction mixture can be achieved by passing an inert gas (e.g. nitrogen) through the reaction mixture and/or the reactants to be fed in or by heating the reactants to be added to the boiling temperature of the reactant in question.

In the process according to the invention various primary amines and/or Schiff bases may be used as the catalyst, e.g., methylamine, ethylamine, n-propylamine, isopropylamine, n-butylamine, isobutylamine, secondary pentyl-amine and/or the Schiff bases of these amines with acetone. In practice the amount of catalyst may be in the range from 0.01 to 0.25 mole of catalyst per mole of methyl acrylate to be converted. In addition to the catalyst, a small amount of an acid or acidic compound must be present in the reaction mixture. An amount of between about 0.01—0.5 moles of acid compound per mole of catalyst, for example, is sufficient. Either organic and inorganic acids are suitable for this purpose, e.g., acetic acid, benzoic acid, adipic acid, hydrochloric acid, phosphoric acid and sulphuric acid.

The amount of acetone used in the process according may be for example from 2 to 15 moles of acetone per mole of methyl acrylate. Larger proportions e.g. 20 moles per mole of methyl acrylate may be used if desired, but little if any practical advantage is thereby attained.

The temperature at which the reaction of acetone with the methyl acrylate is effected may be for example between 75° and 230°C. The pressure is not critical but should be sufficiently high to maintain the reaction mixture in the liquid phase.

The invention will be elucidated in the following example of a continuous type reaction.

Example

Into a stainless-steel 6-litre reactor provided with a stirrer, there was introduced per hour 316 grams of acetone, 479 grams of methyl acrylate, 32 grams of a catalyst mixture comprising 31.0% by weight of isopropylamine, 8.4% by weight of benzoic acid and 60.6% by weight of acetone, and 2343 grams of a recycle mixture obtained as described below and consisting of 0.1% by weight of isopropylamine, 88.5% by weight of acetone, 9.6% by weight of methyl acrylate, and 1.8% by weight of N-isopropylideneisopropylamine. The reaction mixture is passed successively through two other reactors of the same type.

The reaction mixture in the reactors is kept under a nitrogen pressure of about 2000 kPa, while the temperature of the reaction mixture is controlled at about 180 °C by heating the reactors.

The oxygen content of the acetone used is lowered from 50 p.p.m. to 1.5 p.p.m. by passing through nitrogen that is substantially free of oxygen. In the reactors, the oxygen content of the reaction mixture is about 2 p.p.m.

After the reaction mixture has passed the last reactor, it is expanded to atmospheric pressure.

3169 grams of reaction mixture are obtained per hour. The reaction mixture contains 19.5% by weight of the methyl ester of 4-oxopentane-1-carboxylic acid, 7.1% by weight of methyl acrylate, and 65.4% by weight of acetone. The conversion of the methyl acrylate amounts to 68% and that of the acetone to 14%. The yield of methyl ester of 4-oxopentane-1-carboxylic acid is 77% based on the methyl acrylate converted and 74% based on the acetone converted.

The resulting reaction mixture is then fed to a distillation column into which has previously been introduced sufficient of the inert solvent mesityl oxide that the total amount of mesityl oxide in the resulting mixture is about 20% by weight of the amount of methyl ester.

The distillation is effected in a thermally insulated sieve-tray column with an internal diameter of 5 centimeters built up of 25 sieve trays and provided with a reflux cooler, a liquid reflux distributor and a recycle evaporator. The column is fed continuously with 3231 g/h of mixture at the eleventh tray from the top. The top pressure is atmospheric, the bottom temperature is 190 °C and the reflux ratio is 0.5. The distillate (2358 g/h) contains 0.1% by weight of isopropylamine, 87.9% by weight of acetone, 9.6% by weight of methyl acrylate, 1.8% by weight of N-isopropylideneisopropylamine, and 0.7% by weight of water and is recycled. To prevent accumulation of water in the reactors upon recirulation of the distillate, the reaction water formed by reaction of isopropyl amine with acetone to form N-isopropylideneisopropylamine is removed by molecular sieves.

The bottom product (873 g/h) of the distillation column consists of 14.2% by weight of mesityl oxide, 70.8% by weight of the methyl ester of 4-oxopentane-1-carboxylic acid and 15% by weight of residue. This bottom product is fed to a second continuously operated distillation column of the same type as the first except that the feed is at the sixteenth tray from the top, and the column is operated at reduced pressure (top pressure 20 kPa, bottom temperature 160 °C). The reflux ratio is 1 to 2. The distillate (124 g/h) of this column is substantially pure mesityl oxide. Part of it (50% by weight) is recycled to the first distillation column.

The bottom product (749 g/h), consists mainly of the methyl ester of 4-oxopentane-1-carboxylic acid (618 g/h) and residue (131 g/h). This bottom product is fed to a third distillation column of the same type as the first. The column is operated continuously with the feed at the twenty-first tray from the top, a reflux ratio of 3, a top pressure of 2.6 kPa and a bottom temperature of between 215 and 220 °C. The distillate (616 g/h) is the methyl ester of 4-oxopentane-1-carboxylic acid with a purity of 97%. The bottom product contains 10 to 20% by weight of the methyl ester of 4-oxopentane-1-carboxylic acid in addition to residue.

**Claims**

1. Process for preparing the methyl ester of 4-oxopentane-1-carboxylic acid by the liquid phase reaction of acetone with methyl acrylate by using a primary amine and/or Schiff base as a catalyst and in the presence of an acidic compound, in which the unconsumed reactants and the catalyst are first distilled from the resulting reaction mixture and said methyl ester is recovered from the remaining residue by a further distillation, characterized in that the amount of oxygen dissolved in the reaction mixture is maintained at a level below 20 parts by weight per million and the unconsumed reactants and the catalyst are distilled off at a temperature below 195 °C at a pressure not lower than atmospheric in the presence of an inert solvent with a boiling point of between 80 and 195 °C.

2. Process according to claim 1, characterized in that the inert solvent used is mesityl oxide.

3. Process according to claim 1 or claim 2, characterized in that the temperature of the said first distillation is controlled by the amount of inert solvent used.

## Revendications

1. Procédé de préparation de l'ester méthylique de l'acide 4-oxopentane-1-carboxylique moyennant la réaction en phase liquide d'acétone sur le méthylacrylate, en utilisant une amine primaire et/ou une base de Schiff comme catalyseur, en présence d'un composé acide, alors que les réactifs consommés et le catalyseur sont éliminés par une première distillation à partir du mélange réactionnel obtenu et que ledit ester méthylique est récupéré du résidu restant moyennant une autre distillation, caractérisé en ce que la quantité d'oxygène dissoute dans le mélange réactionnel est maintenue au-dessous de 20 parties en poids par million, alors que les réactifs non consommés et le catalyseur sont éliminés par distillation à une température inférieure à 195°C et à une pression qui n'est pas inférieure à la pression atmosphérique, en présence d'un solvant inerte ayant un point d'ébullition situé entre 80 et 195°C.

2. Procédé selon la revendication 1, caractérisé en ce que le solvant inerte utilisé est de l'oxyde de mésityle.

3. Procédé selon l'une des revendications 1 ou 2, caractérisé en ce que la température de ladite première distillation peut être réglée à l'aide de la quantité du solvant inerte utilisé.

## Patentansprüche

1. Verfahren zum Herstellen des Methylesters von 4-Oxopentan-1-Karbonsäure durch die Flüssigphasenreaktion von Aceton mit Methylacrylat unter Verwendung eines primären Amins und/oder einer Schiffschen Base als Katalysator und in Anwesenheit einer sauren Mischung, in der die unverbrauchten Reaktionsmittel und der Katalysator zuerst aus der anfallenden Reaktionsmischung heraus destilliert werden und das der Methylester durch eine weitere Destillation aus dem Rückstand zurückgewonnen wird, dadurch gekennzeichnet, dass die in der Reaktionsmischung gelöste Sauerstoffmenge unter 20 Gewichtsteile je Million aufrechterhalten wird und die unverbrauchten Reaktionsmittel und der Katalysator bei einer Temperatur unter 195 °C und einem überatmosphärischen Druck in Anwesenheit eines inerten Lösungsmittels mit einem Siedepunkt zwischen 80 und 195 °C herausdestilliert werden.

2. Verfahren nach Anspruch 1, dadurch gekennzeichnet, dass das inerte Lösungsmittel Mesityloxid ist.

3. Verfahren nach Anspruch 1 oder 2, dadurch gekennzeichnet, dass die Temperatur der ersten Destillation durch die Menge des inerten Lösungsmittels überwacht wird.